# EUROPEAN PATENT APPLICATION

(11) **EP 4 840 305 A1**
(43) Date of publication of application: **18.11.2026**
(21) Application number: 25177020.2
(22) Date of filing: 16.05.2025
(51) Int. Cl.: A01K 67/30, B65G 15/42, B65G 15/52

(54) **REARING SYSTEM**

(71) Applicant: NBTech AB, 177 32 Järfälla (SE)
(72) Inventor: Berezina, Nathalie, 177 32 Järfälla (SE); BLINOV, Nikita, Ho Chi Minh (VN)
(74) Representative: AWA Sweden AB

(57) **Abstract**

According to a first aspect of the present inventive concept there is provided a rearing system for rearing arthropods, microorganisms, and molluscs. The rearing system comprising a plurality of conveyor belts, configured to convey insects from one conveyor belt to another, wherein the plurality of conveyor belts comprises at least a first conveyor belt having a first total conveying area, and a second conveyor belt having a second total conveying area, wherein the second conveyor belt is arranged below the first conveyor, and wherein the first conveyor belt is configured to convey insects towards a first belt end, and wherein the first belt end is arranged above the second conveyor belt, to thereby drop off insects onto the second conveyor belt, and wherein the second total conveying area is larger than the first total conveying area. According to a second aspect of the present inventive concept, there is provided a rearing system comprising a plurality of substrate delivery units.

## Description

### Technical field

The present disclosure relates to a rearing system, and more particularly to a rearing system for rearing arthropods, microorganisms, and molluscs.

### Background

Rearing insects, also known as insect farming or insect husbandry, involves cultivating insects for various purposes such as food, animal feed, scientific research, or environmental management. This practice is gaining popularity due to its sustainability and low environmental impact compared to traditional livestock farming.

Insects are housed in controlled environments where temperature, humidity, and light are regulated to optimize growth and reproduction.

Prior known solutions for insect-maturation comprises for example tray systems being limited either by high workload or extremely energy consuming HVAC system, or reactors being limited by not being vertical farming systems, therefore requiring larger surface footprint, or a single long conveying belt. While known systems today may offer a degree of automation, they present several limitations, including inefficient use of space, limited environmental control, and challenges in managing insects at different stages of development. Hence, there is still a need for improved rearing systems.

### Summary

An objective of the present disclosure is to provide a more compact and more efficient rearing system for rearing arthropods, microorganisms, and molluscs.

This and other objectives are at least partly met by the invention as defined in the independent claims. Preferred embodiments are set out in the dependent claims.

According to a first aspect, there is provided a rearing system for rearing arthropods, microorganisms, and molluscs.

The rearing system comprises a plurality of conveyor belts, comprising at least a first conveyor belt having a first total conveying area, and a second conveyor belt having a second total conveying area, wherein the second conveyor belt is arranged below the first conveyor, and wherein the first conveyor belt is configured to convey towards a first belt end, and wherein the first belt end is arranged above the second conveyor belt, to thereby drop off onto the second conveyor belt, and wherein the second total conveying area is larger than the first total conveying area.

The system may be used for rearing fairly small organisms having limited movement capacity. For example, small organisms may include arthropods include insects, shellfish, etc., microorganisms include solid state fermentation, and molluscs include for gastropods, e.g., snails.

Throughout the application text the small organisms may be referred to as organisms, a population of organisms or a group of organisms. Insects may be used as a general term, and should here be understood to be able to be interchangeable with arthropods, molluscs and microorganisms. The plurality of conveyor belts may be configured to convey arthropods, molluscs and microorganisms, such as insects.

The rearing system comprises a plurality of conveyor belts. For example, the system may comprise two, three, four, or more conveyor belts. The conveyor belts of the plurality of conveyor belts may be arranged in a stacked manner, such that the first conveying belt, the uppermost, convey, by dropping of, to a second conveyor belt arranged below the first, and the second conveyor belt may convey, by dropping off, to a third conveyor belt arranged below the second conveyor belt. In other words, each subsequent conveyor belt in the plurality of conveyor belts is arranged below the one coming before, except the first and uppermost one.

The number of conveyor belts may be chosen to suit the microorganism chosen for rearing and its life cycle, the space available and/or the total area of the first conveyor belt.

A conveyor belt may be a mechanical system used to transport materials or objects from one point to another along a continuous, or semi-continuous, loop. It may comprise a moving belt stretched between two or more rollers. Each conveyor belt of the plurality of conveyer belts may be straight, i.e. extend in a straight line. The total conveying area of a conveyor belt may herein be defined as the area of a conveyor belt on which something may be conveyed. This may be seen as the total area of the top-side of the conveyor belt, since the surface of the bottom-side cannot hold anything due to gravity. By "arranging below" it may herein mean closer to the source of gravity, i.e. closer to the ground. It is to be understood that a conveyor belt drops off whatever is being conveyed at an end of the conveyer belt, e.g. arthropods, microorganisms, and molluscs, such as insects, and also leftover feed/substrate.

As an example, a certain organism may require twelve days from hatching to be ready to be harvested. During the first four days the organisms are very small in scale, and the rearing therefore takes place in a nursery unit. The remaining eight days, the organisms are resting on the conveyor belts, preferably one belt per day to be able to achieve an optimal environment for effective rearing. This means that in this given example, eight conveyor belts are needed.

In another example, four conveyor belts may be needed, and in a further example the organisms may rest on one conveyor belt for more than a day.

Of the plurality of conveyor belt, each subsequent conveyor belt may be arranged below the preceding conveyor belt. In other words, the second conveyor belt is arranged below the first conveyor belt. Accordingly, if the plurality of conveyor belts comprises more than two conveyor belts, the third conveyor belt is arranged below the second conveyor belt.

Arranging the plurality of conveyor belts in this stacked manner allows for the insects (small organisms) to be moved from a conveyor belt to a subsequent conveyor belt in a more efficient manner, by using gravity. Each conveyor belt may be configured to provide a specific environment, for example, humidity, temperature or a specific substrate, promoting the growth of the organisms at the corresponding development stage. The substrate may be a nutrient-rich organic material suitable for supporting larval development. The substrate may be different things based on what species is being raised, for example, black soldier fly larvae are reared on food waste, mealworms on wheat bran, and crickets on ground grain feed.

It should be appreciated that the present invention provides a solution with a plurality of separate conveying bands which are stacked and, with the help of gravity, allows a batch of insects to move from conveyor belt to conveyor belt, allowing the insects to experience certain conditions for certain times to mature in a controlled manner which is more efficient.

Furthermore, the rearing system, according to the present invention, advantageously provides for a more compact solution, thus being more space- and resource-efficient.

Each conveyor belt may comprise a side wall. A side wall may have a certain height. The cleats and the side walls may together form one or more enclosures for a population of insects, and may prevent escape and may ensure more efficient access to feed.

Furthermore, by having a subsequent conveyor belt with a larger total conveying area than the preceding conveyor belt, the total conveying area may be adapted to a maturation cycle, such as to the size of the insects during maturation, type and/or required amount of fresh substrate, and thus a more space efficient system is provided. For example, a certain population of organisms may increase in size during the conveying from the first conveying belt to the second conveying belt. By providing additional space on the subsequent conveyor belt, unnecessary space is avoided during the maturation on the first conveyor belt, thereby allocating space in a more efficient way. Further, by providing a more optimized area for the insects to mature in as they grow may make the growth process more efficient.

According to some embodiments, the plurality of conveyor belts may comprise a third conveyor belt having a third total conveying area. The third conveyor belt may be arranged below the second conveyor belt, and the second conveyor belt may be configured to convey insects towards a second belt end, and wherein the second belt end may be arranged above the third conveyor belt, to thereby drop off insects onto the third conveyor belt.

The third total conveying area may be larger than the second total conveying area.

Hence, each subsequent conveying belt may comprise a larger total conveying area than the preceding conveyor belt. Thanks to each subsequent conveyor belt having a larger total conveying area than the preceding conveyor belt, the total conveying area may be adapted to a maturation cycle, such as to the size of the insects during maturation, and thus an even more space efficient system is provided. Furthermore, by having three conveyor belts with increased total conveying area, the maturation process may be optimized in at least three different steps, for which the area on which the insects are reached in the three steps may be designed to optimize the rearing process. This provides an even more versatile and improved rearing process.

In a preferred embodiment, the ratio between the total conveying area of the first conveyor belt and the total conveying area of the second conveyor belt may be 1:3, i.e. three times larger area for the second conveyor belt. Further, the ratio between the total conveying area of the second conveyor belt and the total conveying area of the third conveyor belt may be 3:5, i.e. about 67% larger area for the third conveyor belt compared to the second conveyor belt.

According to some embodiments, at least one conveyor belt of the plurality of conveyor belts may comprise a plurality of cleats, arranged spaced apart on the at least one conveyor belt and configured to be moved together with the at least one conveyor belt.

The cleats may be described as divider walls, configured to divide the total area of the conveyor belt. The cleats may have a certain height.

The cleats may be configured to create a plurality of separate areas/compartments of the total conveying area of the conveyor belt. For example, two cleats may create two separate areas of the total area. Preferably, the cleats are evenly spaced out, such that the separate areas are substantially the same or similar in size/area.

The cleats may preferably be arranged parallel to each other. The cleats may be arranged between two sidewalls of the conveyor belt at right angles. It may also be envisioned that the cleats may be arranged diagonally between the side walls.

The enclosures enable each conveyor belt to host a plurality of populations of organisms, one population per enclosure.

The present embodiment is advantageous in that the spread of insects across the total conveying area may be more spread out, in a more uniform manner. Thereby, the rearing may be made more efficient.

According to some embodiments, the first conveyor belt may comprise a first set of cleats, arranged spaced apart on the first conveyor belt with a first separation distance, SP₁, and configured to be moved together with the first conveyor belt. The second conveyor belt may comprise a second set of cleats, arranged spaced apart on the first conveyor belt with a second separation distance, SP₂, and configured to be moved together with the second conveyor belt.

The second separation distance, SP₂, may be greater than the first separation distance, SP₁.

The present embodiment is advantageous in that the spaced areas/comparts by the cleats is adapted to different stages of maturation, such that one population on the first conveyor belt may have more space on a subsequent second conveyor belt, thereby considering the change in size and required volumetric amount of feed during maturation. Thereby the rearing process may be more efficient.

According to some embodiments, the cleats may be evenly spaced apart on the at least one conveyor belt and wherein the insect rearing system is configured to control the speed of the at least one conveyor belt based on the spacing between the cleats.

In other words, the cleats have the same separation distance between each cleat, and the system may control the speed of one or more conveyor belts, and thus the amount of time the insects are resting on the conveyor belt, based on the spacing between the cleats, i.e. the separation distance of the cleats. It is to be understood that the conveying area between the cleats correlates with the separation distance of the cleats. Consequently, the system may be controlled in a manner which is adapted to the characteristics of the cleats, to thereby improve the rearing efficiency.

For example, by knowing the distance between the cleats, the system may be adapted to this, such that the population of insects between cleats are on that conveyor belt for a certain time.

According to some embodiments, at least one conveyor belt of the plurality of conveyor belts may comprise a side wall comprising vent openings configured to allow airflow.

The vent openings may comprise any hole, perforation and/or ducts that allow for the exchange of air between an interior, i.e. the conveying belt and a surrounding environment, i.e. outside the conveying belt.

The conveyor belt may have two side walls on the top side of the conveyor belt, and the vent openings may be arranged on only one side wall or on both side walls. The vent openings may be spaced apart equally along a side wall.

The present embodiment is advantageous in that the conveying belt may be supplied with air via the vent openings, to better reach the insects being conveyed, and thereby increase the efficiency of the rearing process.

In a further embodiment, wherein at least one conveyor belt comprises cleats, that together with the side walls form an enclosure for a population of insects, it may be advantageous that each enclosure comprises at least one vent opening, thereby allowing for airflow in each separate enclosure, to thereby improve the rearing process. In other words, a conveyor belt may comprise a sidewall and cleats, wherein the sidewall has a number of vent openings based on the number of cleats of the conveyor belt.

According to some embodiments, the system may further comprise a ventilation system. The ventilation system may comprise a main ventilation duct, and a plurality of ventilation branches fluidly connected to the main ventilation duct. The main ventilation duct may be configured to direct a flow of air, F1, to the plurality of ventilation branches. The plurality of ventilation branches comprises a first ventilation branch configured to provide a flow of air to the first conveyor belt, and a second ventilation branch configured to provide a flow of air to the second conveyor belt. A ventilation branch may be a secondary ventilation duct fluidly connected to the main ventilation duct.

Good ventilation is important for air quality, temperature, humidity, and hygiene in rearing systems. Without it, insect health, development speed, survival rates, and productivity can be severely compromised. The present embodiment is advantageous in that the rearing system is ventilated in a more efficient manner. By having a ventilation branch, i.e. a ventilation flow path, to each conveyor belt specifically, the rearing process is improved. In a further embodiment, the ventilation branches may be arranged to provide airflow to vent openings in the side-walls of the conveyor belts.

In a further embodiment, the ventilation branches may be arranged to provide airflow to the vent openings in the side-walls of the conveyor belts. This is further advantageous in that the ventilation is improved, and thereby the rearing process.

According to some embodiments, the first total conveying area may comprise a first length, L₁, and a first width, W₁, and the second total conveying area may comprise a second length, L₂, and a second width, W₂.

The second length, L₂, may be greater than the first length, L₁, and/or the second width, W₂, may be greater than the first width, W₁.

The length and/or width may be increased from one conveyor belt to the next in certain intervals. For example, the first conveying area may have a first length, L₁, and the second conveying area may have a second length, L₂, and wherein L₂/ L₁ > 1.15 is fulfilled (meaning that the length is increased by 15% or more to account for the growth).

The first conveying area may have a first width, W₁, and the second conveying area may have a second width, W₂, and wherein W₂/ W₁ > 1.5 is fulfilled (meaning that the width is increased by 50% or more, to account for the growth).

In one example, the system may comprise four subsequent conveyor belts. The total area ratio between the conveyor belts may be 1:3:5:7. In other words, the second conveyor belt may comprise a total area three times the size of the of the total area of the first conveyor belt. The third conveyor belt may comprise a total area 5 times the total area of the first conveyor belt. The fourth conveyor belt may comprise a total area seven times the total area of the first conveyor belt.

It is a realization that the area ratio between the subsequent conveyor belt may be dependent on the developmental stages of the insects. For example, a specific organism may experience a growth surge demanding a higher area ratio.

According to some embodiments, the system may further comprise a control unit. The control unit may be any component configured to monitor, regulate, and/or coordinate the operation of connected devices and subsystems based on predefined parameters or real-time sensor input. The control unit may control the operation of the plurality of conveyor belts, such as speed and start and stop and direction. The control unit may be integrated in the rearing system or be external. The control unit may be configured to control a speed of each of the conveyor belts of the plurality of conveyor belts independently. The present embodiment is advantageous in that the rearing system may control the rearing in a more versatile manner, to make the rearing process more efficient.

According to some embodiments, at least one conveyor belt of the plurality of conveyor belts may be configured to move in two directions. In other words, one or more of the plurality of conveyor belts may be bi-directional. This is advantageous in that some maintenance operations may be improved. Further, this allows more versatility when it comes to operation of the rearing system. In one example, the first conveyor belt (i.e. the uppermost one) is bi-directional. In this case the first conveyor belt may be filled up while moving in one direction, and then drop off to the second conveyor belt below by moving in the other direction. Hence, the present embodiment may provide a rearing system with improved functionality in terms of space usage and/or rearing efficiency.

According to some embodiments, the rearing system further comprises a control unit, wherein the control unit may be further configured to control at least one of the conveying belts to move intermittently at predefined time intervals.

By "moving intermittently" it may here mean that something moves at irregular or repeated intervals, not continuously. Instead of moving in a smooth, constant motion, the conveyor belt may start and stop, or move in short bursts. For example, at least one conveying belt may start moving at a first time, then stop at a later second time, then being still for a third time, and then start moving again at a fourth time.

The present embodiment is advantageous in that the conveyor belts does not have to be activated at all times. This may provide a more energy efficient rearing system, and also provide a more versatile rearing system allowing for different types of conveyor belt movements.

According to some embodiments, the rearing system further comprises a control unit, wherein the control unit may be further configured to control at least one of the conveying belts to move continuously.

It is also to be understood that one or more conveying belts may be controlled to move intermittently or continuously, e.g. controlled by a control unit.

According to some embodiments, the rearing system further comprises a control unit, wherein the control unit may be configured to control the speed of the first conveyor belt and the second conveyor belt according to a predetermined control scheme, wherein the control scheme is based on an insect type and the first total conveying area and the second total conveying area.

Different types of small organisms may mature in different pace, and thus impose different requirements on space, temperature and humidity at different stages and/or timepoints of the maturation process. The rearing system may thus be configured to have conveyor belts with predetermined total conveying areas, and one or more predetermined controls schemes based on the predetermined total conveying areas. Such that the speeds of the conveying bands may be determined based on the total conveying areas and the insect type. The present embodiment is advantageous in that the rearing system is more efficient, versatile and simple to use for an operator.

According to an example, the predetermined control scheme may be further based on a substrate type. The present embodiment is advantageous in that the control schemes are better adapted, to thereby increase the efficiency of the rearing process.

According to some embodiments, the system may further comprise a nursery unit configured to provide the insects to the first conveyor belt, and a control unit configured to control when the nursery unit provides the insects to the first conveyor belt.

The nursery unit may be a dedicated, controlled environment designed to house and care for organisms during their early developmental stages, typically from egg to early larval or juvenile phases, before they are transferred to grow-out or production units, such as the plurality of conveyor belts, more specifically the first conveyor belt .

The present embodiment is advantageous in that rearing system has an integrated nursery unit, providing an even more efficient rearing system, e.g. in terms of space. For example, the control unit may be configured to provide a predetermined amount of insects to the first conveying belt automatically, i.e. without manual user input.

According to some embodiments, the system may further comprise a separating unit, wherein the second conveyor belt is the lowermost conveyor belt of the plurality of conveyor belts, and wherein the second conveyor belt is configured to convey the insects to the separating unit, and wherein the separating unit is configured to receive the insects from the second conveyor belt and to sort the insects.

The separating unit may be a device configured to sort, segregate, and/or isolate insect biomass from non-biological material, or to differentiate between various developmental stages or sizes of insects. This unit may ensure that each component, whether living insect, waste product, or substrate, can be appropriately processed, transferred, or discarded within the rearing workflow.

The present embodiment is advantageous in that rearing process comprises an integrated separating unit, which automatically receives insects from the lowermost conveyor belt, such as the second, third or fourth conveyor belt, or more, depending on the embodiment. The separating unit may be arranged below the lowermost conveyor belt, such that insects are transported by gravity from the lowermost conveyor belt.

According to some embodiments, the first conveyor belt and the second conveyor belt comprises, respectively, at least one of a temperature controller configured to control a temperature of the air around the conveyor belt, and a humidity controller configured to control a humidity of the air around the conveyor belt.

The temperature controller may be configured to monitor and regulate the ambient temperature within the rearing chamber, nursery unit, or associated environment. It may maintain thermal conditions within a predefined temperature range suitable for the targeted insect species' growth and development. The humidity controller may be configured to maintain a relative humidity (RH) within the optimal range for insect egg hatching, larval development, and overall colony health. It may automatically adjust the internal atmosphere by controlling active humidification or dehumidification elements. It is to be understood that each conveyor belt of the plurality of conveyor belts may have a respective temperature controller and/or humidity controller. Further, the temperature controller and the humidity controller may be part of one single environmental control unit.

The present embodiment is advantageous in that the rearing system comprises an integrated temperature controller and humidity controller, which may make the rearing process more efficient.

According to some embodiments, the rearing system comprises a substrate delivery arrangement, wherein the substrate delivery arrangement comprises a first substrate delivery unit configured to deliver substrate to the first conveyor belt, and a second substrate delivery unit configured to deliver substrate to the second conveyor belt.

It is to be understood that each conveyor belt of the plurality of conveyor belts may have its own respective substrate delivery unit. The substrate delivery arrangement may be any component or set of components configured to provide substrate to a conveyor belt. The substrate delivery arrangement may comprise a substrate storage unit, such as a silo or tank or bin. The substrate storage unit may be connected to each substrate delivery unit and configured to deliver substrate to each deliver unit. The substrate delivery unit may be any passage configured to guide and direct substrate, for example a chute, using gravity to deliver substrate to a conveyor belt. The substrate delivery unit may be a conduit, a chute, a channel, a tube, a canal, and/or a duct. The substrate delivery arrangement may comprise one or more flow control devices such as a valve, dumper or similar. For example, each substrate delivery unit may have a corresponding flow control device.

The present embodiment is advantageous in that the substrate may be delivered in a more uniform manner, at different conveyor belts, and thus at different stages of maturation, to make sure the conveyed insects have substrate at more/all stages. Furthermore, it may be advantageous to have a single substrate storage unit connected to a plurality of substrate delivery units which are configured to deliver substrate to two or more conveyor belts, since it is a more efficient way to do it, while also improving the efficiency of the rearing system.

According to a second aspect of the present invention, there is a disclosed a rearing system comprising a substrate delivery arrangement.

This second aspect and its embodiments may have several features in common with the first aspect of the present invention and its embodiments and it is hereby referred to the associated texts of the first aspect. Further, the second aspect may generally present the same or corresponding advantages as the first aspect.

The rearing system comprises a plurality of conveyor belts, comprising at least a first conveyor belt, and a second conveyor belt, wherein the second conveyor belt is arranged below the first conveyor belt, and wherein the first conveyor belt is configured to convey insects towards a first belt end, and wherein the first belt end is arranged above the second conveyor belt, and wherein the insect rearing system further comprises a substrate delivery arrangement, wherein the substrate delivery arrangement comprises a first substrate delivery unit configured to deliver substrate to the first conveyor belt, and a second substrate delivery unit configured to deliver substrate to the second conveyor belt.

The present invention according to the second aspect is advantageous in that substrate may be delivered in a more uniform manner, at different conveyor belts, and thus at different stages of maturation, to make sure the conveyed insects have necessary amount of substrate at more/all stages. Furthermore, it may be advantageous to have a single substrate storage unit connected to a plurality of substrate delivery units which are configured to deliver substrate to two or more conveyor belts, since it is a more efficient way to do it, while also improving the efficiency of the rearing system.

According to some embodiments of the second aspect of the present invention, the first conveyor belt has a first total conveying area, and the second conveyor belt having a second total conveying area, wherein the second total conveying area is larger than the first total conveying area. it is to be understood that the rearing system may comprise any number of conveyor belts, wherein each subsequent/lower conveyor belt has a larger total conveying area compared to the conveyer belt before/above.

According to some embodiments of the second aspect of the present invention, the plurality of conveyor belts comprises a third conveyor belt having a third total conveying area, wherein the substrate delivery arrangement comprises a third substrate delivery unit configured to deliver substrate to the third conveyor belt.

According to some embodiments of the second aspect of the present invention, at least one conveyor belt of the plurality of conveyor belts comprises a plurality of cleats, arranged spaced apart on the at least one conveyor belt and configured to be moved together with the at least one conveyor belt.

According to some embodiments of the second aspect of the present invention, the first conveyer belt comprises a first set cleats, arranged spaced apart on the first conveyor belt with a first separation distance, SP1, and configured to be moved together with the first conveyor belt, and the second conveyor belt comprises a second set of cleats, arranged spaced apart on the first conveyor belt with a second separation distance, SP2, and configured to be moved together with the second conveyor belt, wherein the second separation distance, SP2, is greater than the first separation distance, SP1.

According to some embodiments of the second aspect of the present invention, the cleats are evenly spaced apart on the at least one conveyor belt and wherein the insect rearing system is configured to control the speed of the at least one conveyor belt based on the spacing between the cleats.

According to some embodiments of the second aspect of the present invention, at least one conveyor belt of the plurality of conveyor belts comprises a side-wall comprising vent openings configured to allow airflow.

According to some embodiments of the second aspect of the present invention, the rearing system comprises a ventilation system, wherein the ventilation system comprises a main ventilation duct, and a plurality of ventilation branches fluidly connected to the main ventilation duct, wherein the main ventilation duct is configured to direct a flow of air, F1, to the plurality of ventilation branches, and wherein the plurality of ventilation branches comprises a first ventilation branch configured to provide a flow of air to the first conveyor belt, and a second ventilation branch configured to provide a flow of air to the second conveyor belt.

According to some embodiments of the second aspect of the present invention, the rearing system comprises a control unit configured to control a speed of each of the conveyor belts of the plurality of conveyor belts independently.

According to some embodiments of the second aspect of the present invention, the control unit is further configured to control at least one of the conveying belts to move intermittently at predefined time intervals.

According to some embodiments of the second aspect of the present invention, the control unit is configured to control the speed of the first conveyor belt and the second conveyor belt according to a predetermined control scheme, wherein the control scheme is based on an insect type and the first total conveying area and the second total conveying area.

According to some embodiments of the second aspect of the present invention, the rearing system comprises a nursery unit configured to provide the insects to the first conveyor belt, and a control unit configured to control when the nursery unit provides the insects to the first conveyor belt.

According to some embodiments of the second aspect of the present invention, the rearing system comprises a separating unit, wherein the second conveyor belt is the lowermost conveyor belt of the plurality of conveyor belts, and wherein the second conveyor belt is configured to convey the insects to the separating unit, and wherein the separating unit is configured to receive the insects from the second conveyor belt and to sort the insects.

According to some embodiments of the second aspect of the present invention, the first conveyor belt and the second conveyor belt comprises, respectively, at least one of a temperature controller configured to control a temperature of the air around the conveyor belt, and a humidity controller configured to control a humidity of the air around the conveyor belt.

According to a third aspect, there is a disclosed a rearing system comprising a ventilation system. The rearing system comprises a plurality of conveyor belts, configured to convey insects from one conveyor belt to another, wherein the plurality of conveyor belts comprises at least a first conveyor belt, and a second conveyor belt, wherein the second conveyor belt is arranged below the first conveyor belt, and wherein the first conveyor belt is configured to convey towards a first belt end, and wherein the first belt end is arranged above the second conveyor belt. Furthermore, the rearing system comprises a ventilation system comprising a main ventilation duct, and a plurality of ventilation branches fluidly connected to the main ventilation duct, wherein the main ventilation duct is configured to direct a flow of air, F1, to the plurality of ventilation branches, and wherein the plurality of ventilation branches comprises a first ventilation branch configured to provide a flow of air to the first conveyor belt, and a second ventilation branch configured to provide a flow of air to the second conveyor belt.

This third aspect has several features in common with the first and second aspect of the present invention and it is hereby referred to the associated texts of the first and second aspect for embodiments of the third aspect. Further, the third aspect and its embodiments may generally present the same or corresponding advantages as the first and second aspect.

According to some embodiments of the third aspect of the present invention, at least one conveyor belt of the plurality of conveyor belts may comprise a side wall comprising vent openings configured to allow airflow.

In a further embodiment, the ventilation branches may be arranged to provide airflow to the vent openings in the side-walls of the conveyor belts.

The present invention according to the first, second and third aspect relate to the inventive concept of providing a more efficient rearing system, while being space-efficient. This is achieved through structural and/or functional optimizations that enhance the rearing process, streamline operational logistics, and reduce the space-usage of the system. The invention may enable higher yields per unit volume, facilitate maintenance and monitoring, and reduces labor intensity, while being space efficient. Moreover, the rearing system may be designed to support automated or semi-automated workflows, further increasing throughput and consistency while minimizing human intervention, Thus, the inventive concept may provide both ecological and economic benefits by optimizing production density, lifecycle control, and environmental parameters within a compact infrastructure.

Further objectives of, features of, and advantages with the present invention will become apparent when studying the following detailed disclosure, the drawings, and the appended claims. Those skilled in the art realize that different features of the present invention, even if recited in different claims, can be combined in embodiments other than those described in the following.

### Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.
Fig. 1 is a schematic view of a rearing system according to an embodiment of the present disclosure.
Figs. 2A and 2B are schematic side views of a rearing system according to an embodiment of the present disclosure.
Fig. 3 is a schematic view of a system according to an embodiment of the present invention, as viewed from above.
Figs. 4A and 4B are schematic views of a rearing system according to an embodiment of the present disclosure.

### Detailed description

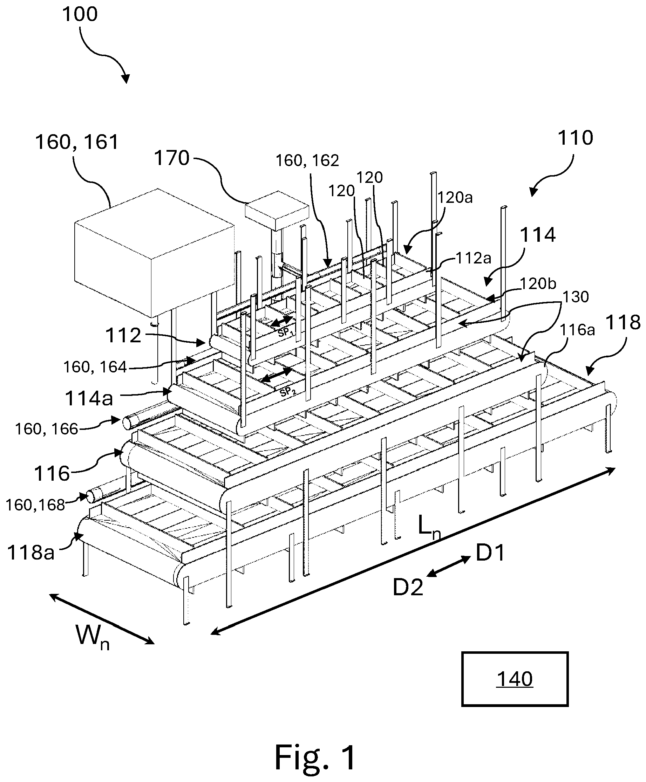

Referring now to Fig. 1, a rearing system 100 for rearing organisms, such as arthropods, microorganisms, and molluscs, will be described.

As illustrated in Fig. 1, the system 100 comprises a plurality of conveyor belts 110. The plurality of conveyor belts 110 is configured to convey organisms, such as arthropods, microorganisms, and molluscs, from one conveyor belt to another. In the illustrated example, a first conveyor belt 112 is configured to convey organisms to a second subsequent conveyor belt 114, by conveying the organisms in a first direction D₁, towards a first belt end 112a. The first belt end 112a is arranged above the second subsequent conveyor belt 114, and thus, as the organisms reach the first belt end 112a they will be conveyed over said end 112a and thereby be dropped onto the second conveyor belt 114. In one example of operation of the rearing system 100, during the filling of the first conveyor belt 112 with substrate the compartments, formed by the cleats 120 and a side wall 130, are filled with substrate consecutively. A first compartment is first filled with substrate, and after filling first compartment the first conveyor belt 112, is moved in a first direction D1. After moving the first conveyor belt 112 the filling of a second compartment is performed. Hence, the filling procedure is repeated until all compartments are filled. The first conveyor belt 112 may then be moved in a second direction D2 at a later stage, e.g. two days later, to drop off to the second conveyor belt 114. The second direction, D₂, is opposite the first direction, D₁. The first conveyor belt 112 may be filled with organisms/insects in different manners, it may be done manually, automatically, or semi automatically. Filling with organisms/insects may be done after filling of the substrate, or at the same time, or before. For example, the first conveyor belt 112 may be manually filled by flipping nursery crates to each compartment, or if the nursery is arranged next to the first conveyor belt 112 and/or above/atop the first conveyor belt 112, an automatic flipper may be implemented. Hence, the rearing system 100 may comprise an automatic flipper configured to transport insects from a nursery unit 150 to the first conveyor belt 112.

It is to be understood that the filling/conveying of the first conveyor belt 112 with substrate and/or organisms may be done in different manners, using movement in one or two directions, and using continuous or intermittent movement. Similarly, the operation of all the conveyor belts may be done in different manners, using movements with different directions and speed and different use of continuous and intermittent movement.

The second subsequent conveyor belt 114 is configured to convey organisms to a third subsequent conveyor belt 116, by conveying the organisms in a second direction D₁, towards a second belt end 114a.

The second belt end 114a may be arranged above a third subsequent conveyor belt 116, and thus, as the organisms reach the second belt end 114a they will be conveyed over said second belt end 114a and thereby be dropped onto the third subsequent conveyor belt 116.

Similarly, the third subsequent conveyor belt 116 may be configured to convey organisms to a fourth subsequent conveyor belt 118, by conveying the organisms in the second direction D₂, towards a third belt end 116a. The third belt end is arranged above the fourth subsequent conveyor belt 118, and thus, as the organisms reach the third belt end 116a they will be conveyed over said third belt end 116a and thereby be dropped onto a fourth subsequent conveyor belt 118.

The fourth subsequent conveyor belt 118 is configured to convey organisms in the second direction D₂, towards a fourth belt end 118a. From the fourth belt end 118a the organisms may be further conveyed to a separating unit (not shown), configured to receive the insects from the last subsequent conveyor belt, here the fourth conveyor belt 118, and to separate and sort the organisms.

Each of the conveyor belt of the plurality of conveyor belts 110 has a total conveying area. The total conveying area of each of the plurality of conveyor belts 110 depends on the length, Lₙ, and the width, Wₙ, of each of the conveyor belts 110. For example, the first total conveying area of the first conveyor belt 112 has a first length, L₁, and a first width, W₁, and the second total conveying area of the second subsequent conveyor belt 114 has a second length, L₂, and a second width, W₂. The second length, L₂, is greater than the first length, L₁. The second width, W₂, is greater than the first width, W₁. Similarly, the third length, L₃, is greater than the second length, L₂, and the third width, W₃, is greater than the second width, W₂.

Each conveyor belt 110 is illustrated as comprising a plurality of cleats 120. The cleats 120 are illustrated as parallel divider walls 120 and arranged spaced apart on each of conveyor belts 110, configured to create separate enclosures on the conveyor belts 110. The conveyor belts 110 further comprises side walls 130, wherein the cleats 120 and the side walls 130 together form an enclosure for a group or population of organisms/insects.

The cleats 120 are further configured to move together with the conveyor belt 110, as can be visualized in Fig. 1 by the cleats 120 being present on the underside of the conveyor belts 110.

In the illustrated example, the first conveyor belt 112 comprises a first set of cleats 120a, arranged spaced apart on the first conveyor belt 112 with a first separation distance, SP1, and configured to be moved together with the first conveyor belt 112.

The second subsequent conveyor belt 114 is illustrated comprising a second set of cleats 120b, arranged spaced apart on the first conveyor belt 112 with a second separation distance, SP₂, and configured to be moved together with the second subsequent conveyor belt 114. The second separation distance, SP₂, is greater than the first separation distance, SP₁.

The system 100 is further illustrated comprising a control unit 140. The control unit 140 may control the speed of the movement of each of the conveyor belts 110. The speed may be controlled by the control unit 140 independently for each of the conveyor belts 110. In other words, the first conveyor belt 112 may move with a first speed, and the second subsequent conveyor belt 114 may move with a second speed, wherein the first speed and the second speed are different from each other, but can be the same.

The control unit 140 may further be configured to control the conveying belts 110 to move intermittently at predefined time intervals. In other words, that each of the conveyor belts 110 has a stepwise movement, moving with the same time intervals or with different time intervals respectively.

The rearing system 100 may further comprise a nursery unit 150 (not shown). The nursery unit 150 is configured to provide the organisms/insects to the first conveyor belt.

The control unit 140 may be configured to control when the nursery unit 150 provides the early-development arthropods, microorganisms, and/or molluscs to the first conveyor belt 112.

The rearing system 100 is further illustrated comprising a substrate delivery arrangement 160 comprising plurality of substrate delivery units , wherein the plurality of substrate delivery units comprises a first substrate delivery unit 162 configured to deliver substrate to the first conveyor belt 112, and a second substrate delivery unit 164 configured to deliver substrate to the second conveyor belt 114. It is further illustrated how the substrate delivery arrangement comprises a third substrate delivery unit 166 and a fourth delivery unit 168. Hence, the substrate delivery arrangement 160 is configured to deliver substrate to each of the plurality of conveyor belts 110. Further, the substrate deliver arrangement 160 comprises a substrate storage unit 161 configured to provide substrate to the substrate delivery units 162, 164, 166. The rearing system 100 comprises a ventilation system 170 with a main air collector, such as a main ventilation duct, and sub-collectors, such as ventilation branches. The ventilation system 170 is used to provide required air flow to each compartment with insects.

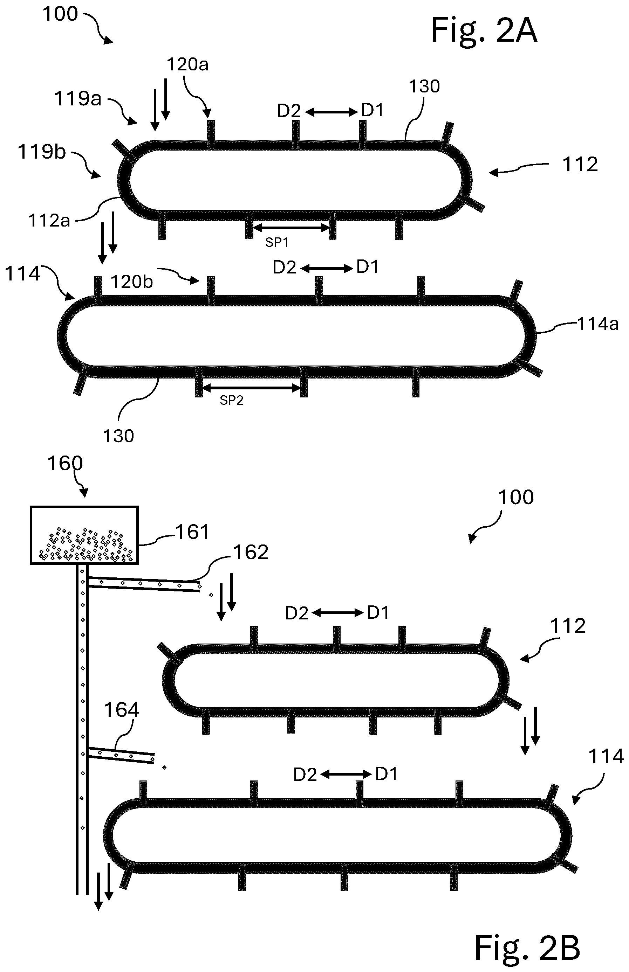

Referring now to Figs. 2A and 2B, a side view of a rearing system 100 for rearing arthropods, microorganisms, and molluscs will be described. Fig. 2 illustrates the system 100 as also described in relation to Fig. 1.

In Figs. 2A and 2B, the rearing system 100 comprises a first conveyor belt 112 and a second conveyer belt 114. The first conveyor belt 112 is configured to convey organisms to a second subsequent conveyor belt 114. In an example of operation, a first compartment 119a is first filled with substrate and organisms, and after filling first compartment 119a the first conveyor belt 112, is moved in a first direction D1. After moving the first conveyor belt 112 the filling of a second compartment 119b is performed. Hence, the filling procedure is repeated until the four compartments are filled. The first conveyor belt 112 may then be moved in a second direction D2 at a later stage, e.g. two days later, to drop off to the second conveyor belt 114.The first belt end 112a is arranged above the second subsequent conveyor belt 114, and thus, as the organisms reach the first belt end 112a they will be conveyed over said end 112a and thereby be dropped onto the second conveyor belt 114.

The second subsequent conveyor belt 114 is configured to convey organisms in a second direction D₂, towards a second belt end 114a. The second direction, D₂, is opposite the first direction, D₁.

In Fig. 2, the first conveyor belt 112 comprises a first set of cleats 120a, arranged spaced apart on the first conveyor belt 112 with a first separation distance, SP₁, and configured to be moved together with the first conveyor belt 112.

The second subsequent conveyor belt 114 is illustrated, comprising a second set of cleats 120b, arranged spaced apart on the second conveyor belt 114 with a second separation distance, SP₂, and configured to be moved together with the second subsequent conveyor belt 114. The second separation distance, SP₂, is greater than the first separation distance, SP₁. By having the second separation distance SP₂ be greater than the first separation distance SP₁ the system 100 allows for and adapts to increased volume of the organisms as they grow during maturation.

Referring now to Fig. 2B, a side view of a rearing system 100 is illustrated. The rearing system 100 in Fig. 2B is similar to the rearing systems 100 of Fig 1 and Fig 2A, and it is hereby referred to Figs 1 and 2A and associated texts for an increased understand of the features and functionality in Fig. 2B. In Fig 2B, the rearing system 100 comprises a first conveyor belt 112 and a second conveyor belt 114 arranged below the first conveyor belt 112. The rearing system 100 further comprises a substrate delivery arrangement 160, comprising a first substrate delivery unit 162, a second substrate delivery unit 165 and a substrate storage unit 161. The first substrate deliver unit 162 is arranged at the first conveyor belt 112, such that the first substrate delivery unit 162 is configured to deliver substrate to the first conveyor belt 112. The second substrate deliver unit 164 is arranged at the second conveyor belt 114, such that the second substrate delivery unit 164 is configured to deliver substrate to the second conveyor belt 114. The first substrate deliver unit and the second substrate delivery unit 164 are connected to the substrate storage unit 161. The substrate storage unit 161 is configured to provide substrate to the substrate delivery units via one or more passages. It is to be understood that the substrate arrangement 160 may comprise one substrate delivery unit for each conveyor belt.

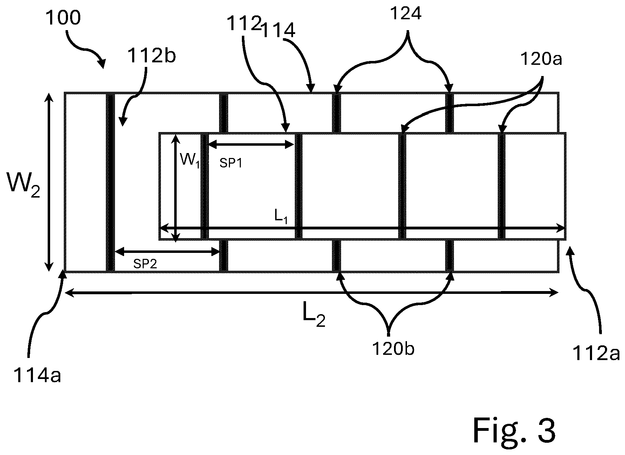

Referring now to Fig. 3, a rearing system 100 according to an embodiment of the present invention, as viewed from above, will be described. The rearing system 100 in Fig. 3 is similar to the rearing system 100 of Fig 1, Fig 2A and Fig 2B, and it is hereby referred to figures 1, 2A and 2B and associated texts for an increased understand of the features and functionality in Fig. 3.

A first conveyor belt 112 is illustrated having a first total conveying area. The first total conveying area has a first length, L₁, and a first width, W₁.

A second conveyor belt 114 is illustrated having a second total conveying area. The second total conveying area has a second length, L₂, and a second width, W₂. The second conveyor belt 114 is arranged below the first conveyor belt 112.

The first conveyor belt 112 is configured to convey insects towards a first belt end, and wherein the first belt end is arranged above the second conveyor belt 114, to thereby drop off insects onto the second conveyor belt 114. In the illustrated example, both belt ends 112a, 112b of the first conveyor belt 112 are arranged above the second conveyor belt 114.

The conveyor belt(s) 112, 114 may be configured to be bidirectional. In other words, each conveyor belt may be configured to convey insects in a first direction and in a second direction.

In the illustrated example, the second total conveying area is larger than the first total conveying area. The second length, L₂, is greater than the first length, L₁, and the second width, W₂, is greater than the first width, W₁.

The first conveyor belt 112 is illustrated as comprising a first set of cleats 120a arranged spaced apart with a first separation distance, SP₁, on the first conveyor belt 112.

The second conveyor belt 114 is illustrated as comprising a second set of cleats 120b arranged spaced apart with a second separation distance, SP₂, on the second conveyor belt 114.

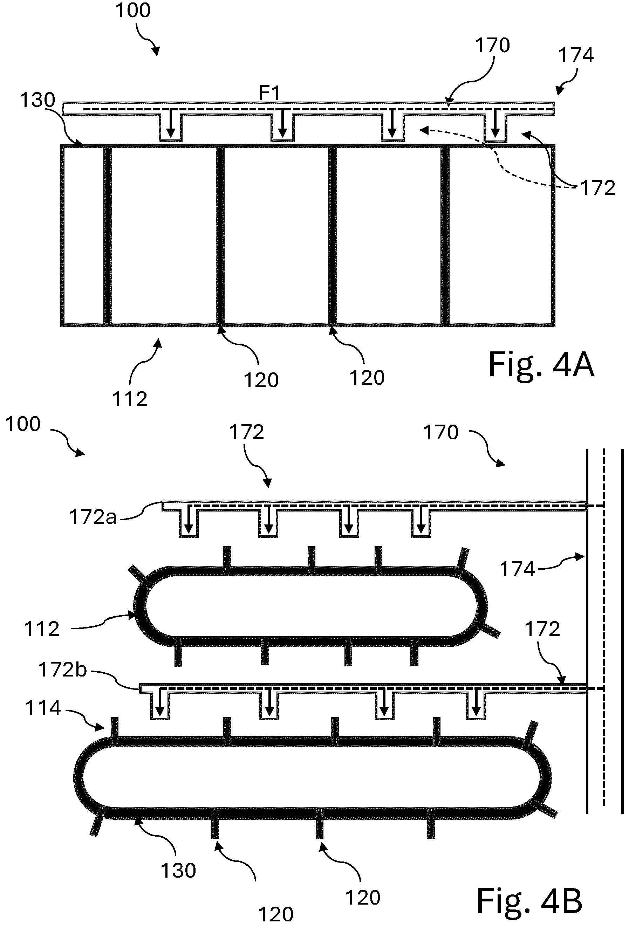

Referring now to Figs. 4A and 4B, a rearing system 100 according to an embodiment of the present disclosure, as viewed from above, Fig 4A, and from the side, Fig 4B. The rearing system 100 in Figs. 4A and 4B is similar to the rearing system 100 of Fig 1, Fig 2A, Fig 2B and Fig 3, and it is hereby referred to Figs 1, 2A, 2B and 3B, and associated texts, for an increased understanding of the features and functionality in Figs. 4A and 4B.

The rearing system 100 comprises a ventilation system 170 comprising a main ventilation duct 174, and a plurality of ventilation branches 172 fluidly connected to the main ventilation duct 174. The main ventilation duct 174 is configured to direct a flow of air, F1, to the plurality of ventilation branches 172. The plurality of ventilation branches 172 comprises a first ventilation branch 172a configured to provide a flow of air to the first conveyor belt 112, and a second ventilation branch 172b configured to provide a flow of air to the second conveyor belt 114. It is to be understood that the ventilation system 170 may comprise a ventilation branch 172 for each conveyor belt. In Fig. 4A and 4B the provided air flow is the same to the first conveyor belt 112 and the second conveyor belt 114. In Figs. 4A and 4B, it can be seen that the ventilation system 170 comprises a ventilation branch 172 for each conveyor belt 112, 114, wherein each ventilation branch 172 has a number of ventilation outputs based on the number of cleats 120 of the respective conveyor belt 112, 114. Here, there are four ventilation outputs, one for each compartment formed by the cleats 120 and side walls 130.

In Fig. 4A, one conveyor belt 112 is shown from above, wherein the conveyor belt 112 has side walls 130, having vent openings through which air from the ventilation branch 172 may pass through to reach the insects on the conveyor belt 112. In Fig 4B, the ventilation branches 172 are illustrated as being arranged above each respective conveyor belt 112, 114, and provides air from above to each conveyor belt 112, 114. However, it is to be understood that the ventilation system 170 may provide air to the conveyor belts 112, 114 from different angles and positions, such as from the side or from above, or both.

Additionally, variations to the disclosed examples can be understood and effected by the skilled person in practicing the claimed invention, from a study of the drawings, the disclosure, and the appended claims. The mere fact that certain measures are recited in mutually different dependent claims does not indicate that a combination of these measures cannot be used to advantage.

A feature described in relation to one aspect may also be incorporated in other aspects, and the advantage of the feature is applicable to all aspects in which it is incorporated. Other objectives, features, and advantages of the present inventive concept will appear from the detailed disclosure, from the attached claims as well as from the drawings.

Generally, all terms used in the claims are to be interpreted according to their ordinary meaning in the technical field, unless explicitly defined otherwise herein. Further, the use of terms "first", "second", and "third", and the like, herein do not denote any order, quantity, or importance, but rather are used to distinguish one element from another. All references to "a/an/the [element, device, component, means, step, etc.]" are to be interpreted openly as referring to at least one instance of said element, device, component, means, step, etc., unless explicitly stated otherwise. The steps of any method disclosed herein do not have to be performed in the exact order disclosed, unless explicitly stated.

## Claims

1. A rearing system (100) for rearing arthropods, microorganisms, and molluscs, the rearing system comprising
a plurality of conveyor belts (110), comprising at least
a first conveyor belt (112) having a first total conveying area, and
a second conveyor belt (114) having a second total conveying area,
wherein the second conveyor belt is arranged below the first conveyor, and wherein the first conveyor belt is configured to convey towards a first belt end (112a), and wherein the first belt end is arranged above the second conveyor belt, to thereby drop off onto the second conveyor belt, and
wherein the second total conveying area is larger than the first total conveying area.

2. The rearing system according to claim 1 or 2, wherein at least one conveyor belt of the plurality of conveyor belts comprises a plurality of cleats (120), arranged spaced apart on the at least one conveyor belt and configured to be moved together with the at least one conveyor belt.

3. The rearing system according to claim 2, wherein
the first conveyer belt comprises a first set cleats (120a), arranged spaced apart on the first conveyor belt with a first separation distance, SP₁, and configured to be moved together with the first conveyor belt, and
the second conveyor belt comprises a second set of cleats (120b), arranged spaced apart on the first conveyor belt with a second separation distance, SP₂, and configured to be moved together with the second conveyor belt,
wherein the second separation distance, SP₂, is greater than the first separation distance, SP₁.

4. The insect rearing system according to claim 2 or 3, wherein the cleats are evenly spaced apart on the at least one conveyor belt and wherein the insect rearing system is configured to control the speed of the at least one conveyor belt based on the spacing between the cleats.

5. The rearing system according to any one of the preceding claims, wherein at least one conveyor belt of the plurality of conveyor belts comprises a side-wall comprising vent openings configured to allow airflow.

6. The rearing system according to any one of the preceding claims, further comprising a ventilation system (170), wherein the ventilation system comprises
a main ventilation duct (174), and
a plurality of ventilation branches (172) fluidly connected to the main ventilation duct,
wherein the main ventilation duct is configured to direct a flow of air, F1, to the plurality of ventilation branches, and
wherein the plurality of ventilation branches comprises a first ventilation branch (172a) configured to provide a flow of air to the first conveyor belt, and a second ventilation branch (172b) configured to provide a flow of air to the second conveyor belt.

7. The rearing system according to any one of the preceding claims, wherein the first total conveying area has a first length, L₁, and a first width, W₁, and the second total conveying area has a second length, L₂, and a second width, W₂, wherein
the second length, L₂, is greater than the first length, L₁, and/or
the second width, W₂, is greater than the first width, W₁.

8. The rearing system according to any one of the preceding claims, wherein at least one conveyor belt of the plurality of conveyor belts may be configured to move in two directions.

9. The rearing system according to any one of the preceding claims, further comprising a control unit (140), wherein the control unit is further configured to control at least one of the conveying belts to move intermittently at predefined time intervals.

10. The rearing system according to any one of the preceding claims, further comprising a control unit (140), wherein the control unit is configured to control the speed of the first conveyor belt and the second conveyor belt according to a predetermined control scheme, wherein the control scheme is based on an insect type and the first total conveying area and the second total conveying area.

11. The rearing system according to any one of the preceding claims, further comprising
a nursery unit (150) configured to provide the insects to the first conveyor belt, and
a control unit (140) configured to control when the nursery unit provides the insects to the first conveyor belt.

12. The rearing system according to any one of the preceding claims, further comprising
a separating unit, wherein the second conveyor belt is the lowermost conveyor belt of the plurality of conveyor belts, and wherein the second conveyor belt is configured to convey the insects to the separating unit, and wherein the separating unit is configured to receive the insects from the second conveyor belt and to sort the insects.

13. The rearing system according to any one of the preceding claims, wherein the first conveyor belt and the second conveyor belt comprises, respectively, at least one of
a temperature controller configured to control a temperature of the air around the conveyor belt, and
a humidity controller configured to control a humidity of the air around the conveyor belt.

14. The rearing system according to any one of the preceding claims, wherein the rearing system comprises a substrate delivery arrangement (160), wherein the substrate delivery arrangement comprises a first substrate delivery unit (162) configured to deliver substrate to the first conveyor belt, and a second substrate delivery unit (164) configured to deliver substrate to the second conveyor belt.

15. A rearing system (100) for rearing arthropods, microorganisms, and molluscs comprising
a plurality of conveyor belts (110)comprising at least
a first conveyor belt (112), and
a second conveyor belt (114),
wherein the second conveyor belt is arranged below the first conveyor belt, and wherein the first conveyor belt is configured to convey towards a first belt end (112a), and wherein the first belt end is arranged above the second conveyor belt, and
wherein the rearing system further comprises a substrate delivery arrangement (160), wherein the substrate delivery arrangement comprises a first substrate delivery unit (162) configured to deliver substrate to the first conveyor belt, and a second substrate delivery unit (164) configured to deliver substrate to the second conveyor belt.
